Europäisches Patentamt

**(19)** European Patent Office

Office européen des brevets

**(11)** **EP 0 661 960 B1**

**(12)** **EUROPEAN PATENT SPECIFICATION**

**(45)** Date of publication and mention
of the grant of the patent:
**06.08.1997 Bulletin 1997/32**

**(21)** Application number: **93915163.5**

**(22)** Date of filing: **28.05.1993**

**(51)** Int. Cl.$^6$: **A61F 13/58**

**(86)** International application number:
**PCT/US93/05139**

**(87)** International publication number:
**WO 94/06388 (31.03.1994 Gazette 1994/08)**

**(54) LOW NOISE DISPOSABLE DIAPER FASTENING TAPE**

GERÄUSCHARMES KLEBEVERSCHLUSSBAND FÜR EINE WEGWERFWINDEL

BANDES DE FIXATION DE COUCHES JETABLES FAISANT PEU DE BRUIT LORSQU'ELLES
SONT DETACHEES

**(84)** Designated Contracting States:
**BE DE ES FR GB IT NL SE**

**(30)** Priority: **24.09.1992 US 950204**
**24.09.1992 JP 254661/92**

**(43)** Date of publication of application:
**12.07.1995 Bulletin 1995/28**

**(73)** Proprietor:
**MINNESOTA MINING AND MANUFACTURING C
OMPANY**
**St. Paul, Minnesota 55133-3427 (US)**

**(72)** Inventors:
• **MILLER, John, A.**
**Saint Paul, MN 55133-3427 (US)**

• **VELASQUEZ UREY, Ruben, E.**
**Saint Paul, MN 55133-3427 (US)**
• **KONO, Yasuhiro**
**Saint Paul, MN 55133-3427 (US)**
• **TATE, Earl, Jr.**
**Saint Paul, MN 55133-3427 (US)**
• **AKIYAMA, Yoshihiro**
**Saint Paul, MN 55133-3427 (US)**
• **MULDER, Robert, S.**
**Saint Paul, MN 55133-3427 (US)**

**(74)** Representative: **VOSSIUS & PARTNER**
**Postfach 86 07 67**
**81634 München (DE)**

**(56)** References cited:
**EP-A- 0 306 232**      **WO-A-92/07042**
**WO-A-92/08763**

**Description**

The present invention relates to a disposable diaper closure system in which the fastening tape makes little noise when detached from the diaper.

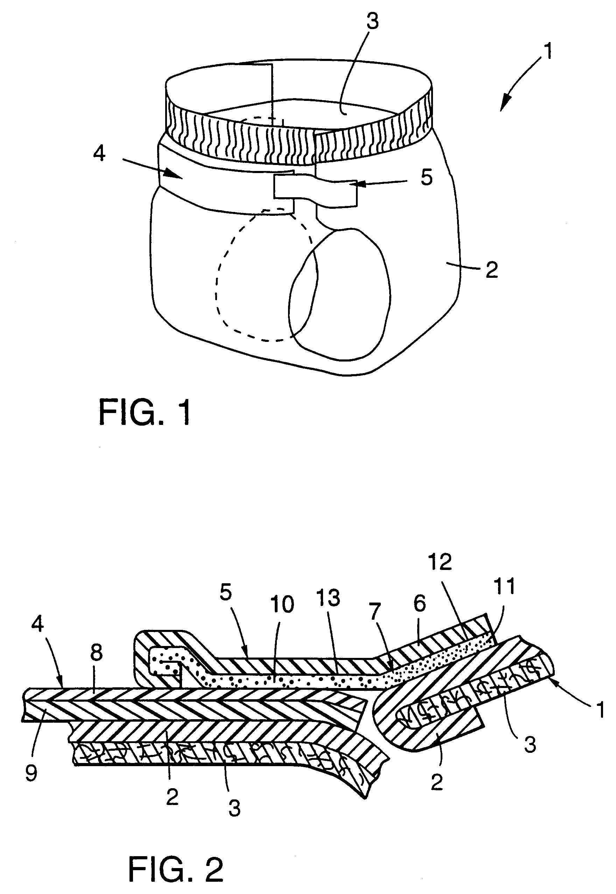

Disposable diapers hitherto used are composed of a body of the diaper, produced by overlapping a non-water-permeable backsheet 2 and a water-permeable interior sheet 3 which encase an absorbent batt between them at least in a central region. A frontal tape 4 is adhered on one part (one end) of said backsheet 2 and a fastening tape 5 is adhered over the surface of said frontal tape and another part (the other end) of said backsheet 2 to close the diaper, one example of which is as shown in Fig. 1.

U.S. patent 5,024,672 discloses a diaper from which a fastening tape can be peeled off without rupturing or tearing the backsheet by laminating a film such as a polypropylene film to the backsheet.

Japanese Unexamined Patent Publication (Kokai) No. 59228008 discloses a diaper with permeability improved by using a foamed product as a part of the frontal portion. Also, Japanese Unexamined Patent Publication (Kokai) No. 6477604 discloses a diaper in which the frontal tape is omitted and a release treatment is directly applied onto the backsheet of the diaper. However, these specifications do not concretely describe a specific adhesive. That is, they do not confront the problem of the harsh granting noise or shockiness as by separation of the frontal tape portion of the diaper and the fastening tape and, thus do not teach any means for solving such a problem.

There are numerous patents and literature documents that are directed to the use of block copolymers in adhesive compositions which traditionally include a block copolymer such as an A-B-A block copolymer and a tackifying resin, for example U.S. Patent No. 3,427,269 (Davis), U.S. Patent No. 3,932,328 (Korpman), U.S. Patent No. 3,954,692 (Downey), U.S. Patent No. 4,097,434 (Coker), U.S. Patent No. 4,460,364 (Chen et al.)and U.S. Patent No. 3,935,338 (Robertson et al.).

EP-A-510 200 (WO-A-92/07042) discloses a structure with a member such as a tape which is difficult to detach while being fixed and can be peeled off without making any noise. The boundary of the release part is composed of a pressure sensitive adhesive layer and a silicone releasant layer.

WO-A-92/08763 refers to a tape closure system exhibiting high shear and high non-shocky peel to a Low Adhesion Backsize coated substrate. The adhesive used on the fastening tape used in the closure system comprises a high diblock ($\geq$ 25 percent) styrene-isoprene-styrene elastomer component with a composite midblock glass transition temperature of 244 to 264 Kelvin with 30 to 60 percent elastomer.

Although there exists extensive art on the use of block copolymers in PSA compositions, none address the problem of lowering the noise of removing a diaper fastening tape from a frontal tape.

The present invention is directed at providing a disposable diaper closure system where the noise caused by separating the fastening tape from the frontal tape portion of the diaper is suppressed.

The present inventors have made a serious study of the problem described above. As a result, it has been discovered that the problem described above can be solved by a combination of a release agent to be coated on the surface of the frontal tape and a specific adhesive for the fastening tape, thereby the present invention having been accomplished.

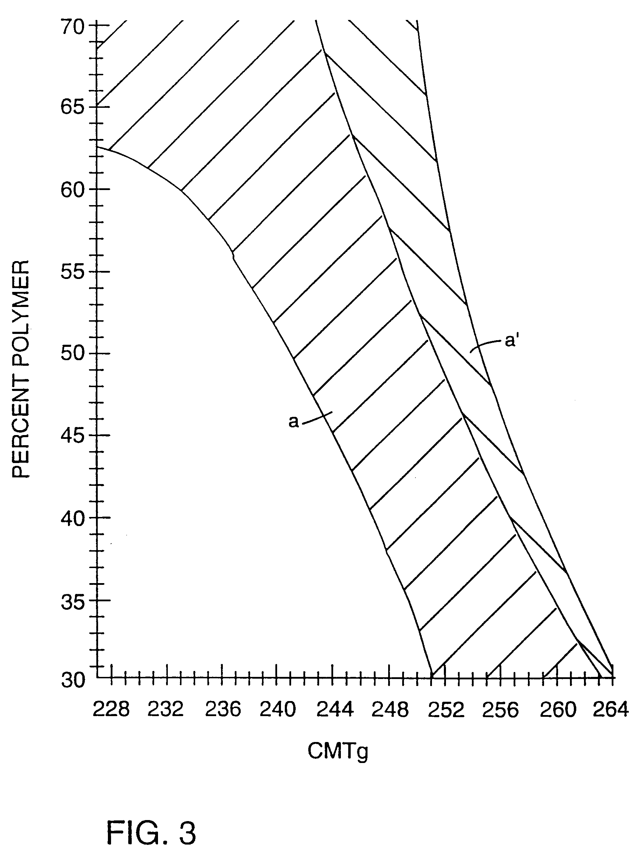

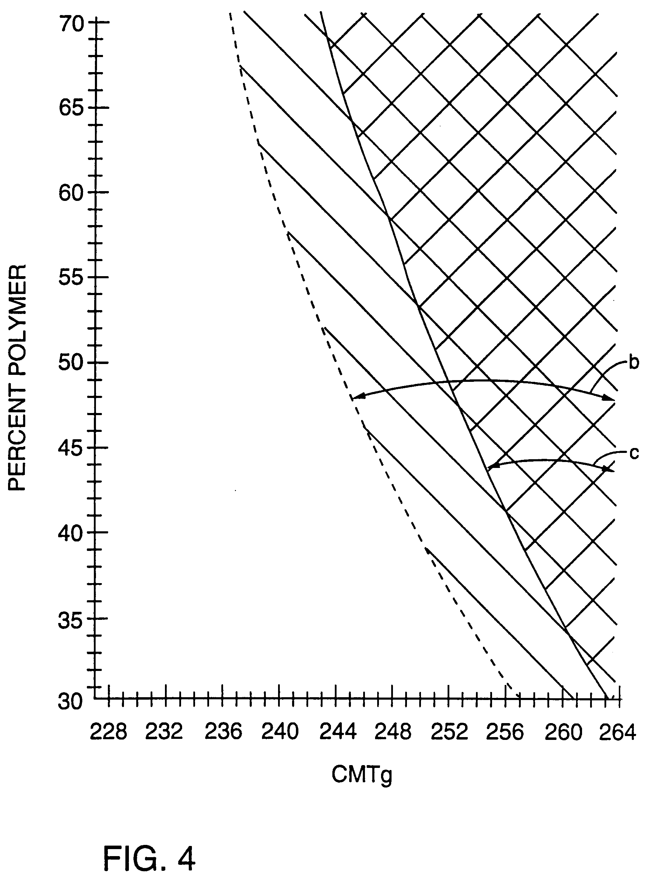

Therefore, the present invention is to provide a disposable diaper having a closure system in which a frontal tape or film is adhered on one end of a backsheet of the body of the diaper, and the diaper is worn in a manner where a fastening tape attaching surface is applied onto the surface of said frontal tape or film and a fastening tape bonding surface is applied over another end of the backsheet, characterized in that a release agent is coated on the surface of the frontal tape or film opposite the surface which is attached to the backsheet, and an attaching adhesive, which comprises an A-B type block copolymer and a tackifier with the glass transition point of the adhesive being in the range of 230K to 260K, and falling within region $\underline{a}$ of Fig. 3, is applied on the attaching surface of said fastening tape. A bonding adhesive is applied to the bonding surface of said fastening tape which preferably falls within region (b) and/or (c) of Fig. 4.

A first preferred attachment adhesive composition comprises:

(a) 30 to 60 weight percent of an elastomeric A-B-(A) block copolymer of at least one polystyrene block A and at least one polyisoprene block B, wherein the A blocks comprise from 10% to 30% of the copolymer and at least 25 percent of said A-B block copolymer comprises an A-B diblock copolymer with the balance comprising an A-B-A block copolymer, and

(b) a mixture of solid tackifying resin, liquid tackifying resin and/or plasticizing oil so as to provide a composite midblock glass transition temperature of from about 264 to 244 Kelvin, which adhesive composition exhibits a non-shocky peel and a 135° peel strength of greater than approximately 300 grams per inch (as defined herein) to a release agent coated film substrate, and a shear strength of at least 100 minutes (as defined herein) to the same substrate.

A second preferred attachment adhesive comprises

2

(a) 45 to 95 weight percent of an elastomer component comprised of a block copolymer with A blocks derived primarily from styrene and B blocks derived primarily from isoprene wherein-at least 15 percent of the block copolymer is in the form of an A-B diblock copolymer,

(b) 5 to 55 weight percent of a solid B block compatible tackifying resin admixed when necessary with a liquid tackifying resin or a processing oil so as to provide an adhesive composition having a CMTg of from 230 to 244 Kelvin which attaching adhesive layer on said fastening tape exhibits a shear adhesion of at least about 100 minutes to the LAB-coated frontal film backing face and a substantially nonshocky peel at 125 degrees of at least about 200 grams per 2,54 cm (inch).

Fig. 1 is a diaper using the closure system of the invention type.

Fig. 2 is an enlarged cross-sectional view of a diaper closure system of the invention.

Fig. 3 is a diagram outlining the preferred attachment adhesive compositions of the invention based on percent elastomer (Y-axis) and adhesive CMTg (X-axis).

Fig. 4 is a diagram outlining the preferred bonding adhesives of the invention as per Fig. 3.

One example of a disposable diaper construction applying in the present invention closure system is explained by referring to Fig. 1. The characteristics of the present invention reside in a release agent or low adhesion back size (LAB) applied on the outer surface of the reinforcement frontal tape 4 and the attaching and bonding adhesives on the fastening tape and, thus, the present invention is not specifically restricted to a specific construction or diaper shape. In Fig. 1, the body 1 of the diaper is constructed by overlapping a non-water-permeable backsheet 2 and a water-permeable interior sheet 3. With regard to the backsheet 2, materials which are conventionally utilized as materials for backsheets of disposable diapers are used. For instance, a usual polyethylene film, a microporous polyethylene film, laminates of these films with nonwovens or the like can be used. The water-permeable interior sheet 3, can be conventionally used interior sheets for diapers, for instance, a non-woven fabric such as made of polypropylene, polyethylene or polyester can be used.

Fig. 2 shows the frontal tape 4 on a first backsheet end and the second backsheet 2 end secured by using the fastening tape 5. In this figure, the fastening tape 5 is composed of a tape substrate 6 and an adhesive layer 7 applied on one side thereof. The frontal tape or film 4 comprises a tape substrate 9, and a release layer or LAB 8 applied on the surface thereof. Further, the body of the diaper comprises a backsheet 2 and an interior sheet 3.

On the right side of Fig. 2, the backsheet 2 and a fastening tape substrate 6 are adhered by means of a bonding adhesive layer 11 on a bonding surface 12 of tape substrate 6. The fastening tape 5 is adhered to the frontal tape or film 4 by means of attaching adhesive layer 10 on attaching surface 13 and is detachable due to the existence of the release layer or LAB 8 on the frontal tape backing substrate 9.

As the substrate 9 of the reinforcing frontal tape or film, a conventional film such as an oriented polypropylene film (OPP), e.g., biaxially oriented polypropylene (BOPP), or a non-oriented polypropylene film (CPP), or a polyester film (PET) is used, the thickness of which is preferably 10 to 50 $\mu$m. Substrate 6 of the fastening tape 5 can be formed of films of polypropylene, polyethylene, mixtures thereof, paper, or the like, the thickness of which is preferably 50 to 200 $\mu$m.

The release agent used in the present invention can be, for example, a urethane, polysiloxane-polyurea or a silicone type release agent. A preferred silicone type release agent is one which comprises a silicone release agent and a release-controlling agent. As the silicone release agent, an addition-reaction type silicone release agent is especially preferable due to its high reactivity. Typically, the silicone release agent comprises mainly polydimethylsiloxane having vinyl groups, for example, SD-7234 (Toray Dow Corning), KS-7764 (Shinetsu Chemical) KS-830E (Shinetsu Chemical), etc. can be used.

A release-controlling agent can be used for the purpose of increasing the peel strength of adhesives to the release coated 8 surface 9, and, for instance, can comprise mainly hydromethylsiloxane, MQ resin, meaning a resin having M units ($(CH_3)_3SiO_{1/2}$) and Q units ($SiO_{4/2}$), or MQ resin derivatives. For example, BX-24-312 (Toray Dow Corning), X-92-140 (Shin-et-su Chemical), etc. can be used.

The proportion of silicone release agent to the release-controlling agent, varies depending on the particular release agent, however, preferably there is used 20-200 parts by weight of the release controlling agent, based on 100 parts by weight of the silicone release agent. If the release controlling agent exceeds 200 parts by weight, release controlling agent remains unreacted, which leads to a decrease in the adhesion strength of the adhesive. Conversely, if the release controlling agent is less than 20 parts by weight, the peel strength of the adhesive is insufficient.

A catalyst for curing the above-mentioned silicone-type release agent can be a platinum catalyst, a tin type catalyst, an acid catalyst, or the like. The platinum catalysts include SPX-212 (Toray Dow Corning), PL-50-T (Shin-et-su Chemical), and the like. When the silicone type release agent is applied on the frontal tape or film, the abovementioned components are dissolved in a solvent, e.g., toluene, benzene, heptane, etc. or a mixture thereof. One example of a preferable solvent mixture is a 1:1 (weight ratio) mixture of toluene and heptane.

The total concentrations of the above components in the solvents is in the range of 1 to 10 parts by weight, preferably 2 to 5 parts by weight, for example, 3 parts by weight, based on 100 parts of the solvent.

This release agent solution is applied on one side of the frontal tape by a conventional means such as a gravure coater, or a roll coater. The amount to be applied is 0.1 to 1.0 g/m$^2$. After the application, any required curing treatment is carried out by a conventional curing means, such as heating or ultraviolet irradiation, depending on the components.

Preferred polysiloxane polyureas are those such as described in EP-A-250 248 and EP-A-380 236 where a segmented block copolymer of the (AB)$_n$ type is produced by condensation polymerization of an essentially pure difunctional organopolysiloxane diamine soft segment with a diisocyanate and an optional chain extender such as a difunctional amine or alcohol soft or mixture, as soft segments. The silicone diamine segment can have a molecular weight of about 1,000 up to 100,000 or more, preferably 2,000 to 50,000. The silicone diamine soft segment is at least 1 percent of the polymer, preferably 2 to 15 percent, after 15 percent the release values change relatively little and use of high percentages would be uneconomical. The diisocyanate hard segment can comprise from 30 to 90 weight percent of the polymer, preferably 40 to 80 percent, with at least 15 weight percent non-silicone soft segment, preferably 20 to 35 weight percent, with at least 20 percent total soft segment. At a weight percent of less than 15 to 20 percent non-silicone soft segment, the release coating becomes more noisy. At greater than 40 percent non-silicone soft segment, the material becomes unstable at typical box car temperatures greater than 65°C. The ratio of silicone to non-silicone soft segment is generally 1:60 to 4:1.

Two important aspects of fastening tape performance in a diaper, or the like, adhesive closure system are the shear resistance and peel strength performance. Peel strength is important in terms of adhesive fastening tape performance. A low peel strength bond increases the risk of the fastening tape 5 popping open when subjected to the forces encountered during use. Further, low peel strengths are often associated with shocky or noisy peels (generally tested at a peel rate of 30.5 cm (12 inches) per minute). Shocky peels are well understood in the art and are when the fastening tape 5 peels in a jerky and noisy (sounding somewhat like a zipper) manner.

The fastening tape adhesive used in the present invention comprises an A-B type block copolymer and a tackifier. The A-B type block copolymer is represented by the following formula:

$$A\text{-}(B\text{-}A)n \text{ and } A\text{-}B$$

wherein A is derived from a vinyl aromatic hydrocarbon, preferably styrene or styrene derivatives, B is derived from a conjugated diene aliphatic hydrocarbon, preferably isoprene, and n is an integer of one or more, with preferably at least 15% of the block copolymer an A-B or diblock copolymer, and most preferably at least 25% and from 30 to 65% for a first preferred adhesive, and from 40% up to about 95% A-B diblock copolymer for a second preferred adhesive.

The A block has a monalkenyl arene, mainly polystyrene, has a molecular weight between 4,000 and 50,000, preferably between 7,000 and 30,000. The A block content is from about 10 to 50 percent, more preferably between 10 and 30 percent. Other suitable A blocks may be formed from alphamethyl styrene, t-butyl styrene and other ring alkylated styrenes as well as mixtures thereof. The B elastomeric conjugated diene, namely isoprene, has an average molecular weight of from about 5,000 to about 500,000, preferably from about 50,000 to 200,000. Although preferably A-B-A and A-B block copolymers will comprise the majority of the elastomer of the adhesive, other conventional diene elastomers may be used to a minor extent, such as natural rubber; butadiene, isoprene or butadiene-styrene rubber; butadiene-acrylonitrile; butyl rubber or block copolymers of these diene elastomers. The block copolymer is used in an amount ranging from about 30 to 60 weight percent, preferably at least 35 weight percent of the adhesive composition.

The tackifying resin component generally comprises a blend of a solid tackifying resin and a liquid tackifying resin, a single solid or liquid tackifying resin, or a blend of a solid tackifying resin and a liquid plasticizer and/or liquid tackifying resin. The tackifying resins can be selected from the group of resins at least partially compatible with the B blocks of the elastomeric materials of this invention. Such tackifying resins include those aliphatic hydrocarbon resins made from the polymerization of a feed stream consisting mainly of unsaturated species containing four to six carbon atoms; rosin esters and rosin acids; mixed aliphatic/aromatic tackifying resins; polyterpene tackifiers; and hydrogenated tackifying resins. The hydrogenated resins can include resins made from the polymerization and subsequent hydrogenation of a feedstock consisting mostly of dicyclopentadiene; resins produced from the polymerization and subsequent hydrogenation of pure aromatic feedstocks such as styrene, alphamethylstyrene, vinyl toluene; resins fashioned from the polymerization and subsequent hydrogenation of an unsaturated aromatic feedstream wherein the feedstream mainly contains species having from 7 to 10 carbon atoms; hydrogenated polyterpene resins; and hydrogenated aliphatic and aliphatic/aromatic resins. Preferred tackifying resins include the aliphatic hydrocarbon resins and the hydrogenated resins. Especially preferred are the aliphatic hydrocarbon resins.

The liquid plasticizers suitable for use in the adhesive compositions of this invention include naphthenic oils, paraffinic oils, aromatic oils, and mineral oils. Preferred plasticizing liquids include naphthenic oils and slightly aromatic oils.

The adhesive preferably is tackified with a solid tackifying resin with a liquid plasticizer of liquid resin of the above described preferred types.

Preferably, the solid tackifying resin used is one that is compatible with the elastomeric conjugated diene block and having a softening point between 80 and 115°C, such as is produced from polymerization of a stream of aliphatic petroleum derivatives of dienes and monoolefins having 4 to 9 carbon atoms as is disclosed in U.S. Patent Nos. 3,932,328 and 3,954,692. Particularly preferred are tackifying resins resulting from the copolymerization of a feed comprised predominately of $C_5$ carbon atom species such as piperylene and 2-methyl-2-butene or isoprene, commercially available, for example, as Wingtack™95 and Wingtack™Plus, respectively, from Goodyear Chemical Co.

The adhesive compositions can also be modified with well known additives such as pigments, fillers, stabilizers and antioxidants for their conventional purposes.

The composite glass transition point (CMTg) of the B phase of the attaching adhesive of the present invention is required to be adjusted to 230 to 260K, and will generally fall within region a of Fig. 3, which is attained by varying the types of A-B type block copolymer and tackifier with any added plasticizing oil and selecting a composition ratio thereof.

If the composite glass transition point of the B phase is lower than 230K or below region a, although the noise made on peeling off is low, the adhesion strength between the fastening tape and the frontal tape is not sufficient; thus this is not practical. If the composite glass transition point of the B phase is higher than 260K or above region a, although the adhesion strength between the fastening tape and the backsheet is sufficient, the noise made on peeling off cannot be satisfactorily suppressed even when the adhesive of the present invention is used. In the present invention, although the glass transition point of the A phase is not specifically restricted, it is preferred to be not less than 50°C in order to not lower the retensivity.

The CMTg can be calculated using the Fox Equation from measuring the Tg of the midblock of the elastomeric block copolymer and the measured Tg of each tackifying resin and liquid plasticizer oil. The Tg for each component is measured using a differential scanning calorimeter such as a DSC-7, manufactured by Perkin-Elmer. The Tg is measured on the second heating run using a scan rate of 20 degrees Centigrade per minute. The first heating run is made up to well above the softening point of the test material. The sample is subsequently quenched to well below the Tg of the material. Antioxidants added to the adhesive are not figured into the calculation of the CMTg. The Fox Equation is:

$$\frac{\Sigma_i W_i}{CMTg} = \Sigma_i \frac{W_i}{Tg_i}$$

where $W_i$ is the weight fraction of component i and $Tg_i$ is the glass transition temperature or component i. Only the midblock portion of the block copolymer is included in the calculation of the CMTg. For a styrene/isoprene block copolymer, the midblock is the polyisoprene portion of the molecule.

The adhesive fastening tab is used in conjunction with a release tape, where the fastening end of the fastening tab is placed prior to attachment to the frontal reinforcing film or tape. The release tape can be of conventional design. One side will have a pressure-sensitive adhesive coating for adhering it preferably to the nonwoven inner liner of the diaper. The opposing side will be coated with a release agent such as crosslinked poly(dimethyl-siloxane).

The bonding adhesive for the fastening tape also preferably comprises an A-B type block copolymer and a tackifier as described above. In this manner, the adhesives are compatible in terms of coating manufacturability. However, the adhesive will generally fall within region b of Fig. 4, and preferably region c. At CMTg values for the bonding adhesive of greater than 275 Kelvin, adhesion to diaper polyethylene becomes poor. Below region b, the adhesive has low initial holding power to polyethylene and moderate holding power in region b outside of region c. However, low adhesion to diaper polyethylene is desirable for an attaching surface adhesive as it will tend not to tear the diaper if the attaching surface is accidentally adhered to the polyethylene.

The adhesive coating thickness applied is generally 20 to 100 μm preferably 25 to 50 μm. The fastening tape thus prepared is cut into a prescribed length, and approximately half the length thereof is placed on a predetermined portion of the backsheet. The remaining portion of the fastening tape is adhered to the surface of the release layer of the frontal tape in use and to a release tape prior to use.

The first preferred adhesive fastening tapes of this invention exhibits consistently high peel values to LAB or release treated substrates, e.g., at least about 300 grams per inch to a common urethane LAB treated polyolefin film, preferably a peel of at least 350 g/2.54 cm (in.) Further, the first preferred adhesive fastening tape with its high elastomeric diblock component has consistently exhibited non-shocky peels to release or LAB treated polyolefin films.

The shear force resistance for a commercial adhesive fastening tab is preferably at least 200 minutes and more preferably at least 300 minutes with a 1 kilogram weight. Shear resistances of less than 300 down to about 100 are still nominally functional yet are not commercially desirable. Shear values much greater than 500 contribute little added functional or commercial benefit to a diaper fastening tab.

The first preferred adhesive has a CMTg of greater than 244 Kelvin. The adhesive compositions falling at the upper edge of region a have been found to provide superior performance as to both smooth and matte finished release coated frontal tape or film. Providing smooth peels or substantially non-shocky peels, high peel strengths, and good or adequate shear resistance performance with the preferred tackifiers.

With higher diblock content elastomers (e.g., greater than 45 percent) adhesives exhibit improved non-shocky peel at higher CMTg values, for a given percent elastomer. As such, the upper limit on peel performance in region a will shift slightly to the right.

A second preferred embodiment of the invention is for adhesives having a composite midblock glass transition temperature below 244 Kelvin to about 230 Kelvin. It has been found that such adhesives can provide very smooth or non-shocky peel performance, however, at a reduction in peel performance as one approaches lower CMTg values until about 230 Kelvin, at which point peel performance generally becomes unacceptable. In this lower CMTg region, the shear performance is lower except at high polymer levels. The lowering of shear performance is also noted against polyethylene film. However, this is advantageous as these tapes are less likely to tear a thin polyethylene backsheet if accidently adhered to the backsheet. Functional adhesives are obtainable with a polymer weight percent of from about 45 to 95 percent, preferably 45 to 80 percent.

The CMTg values described above, although an excellent predictor of peel behavior for a given percent diblock adhesive composition, may not adequately predict shear performance with respect to certain non-preferred tackifiers and tackifying systems or release coatings. For these systems, shear can be raised within the teachings of this invention by increasing the elastomer and/or solid resin content of the adhesive composition used within the outlined CMTg ranges.

A further aspect of the adhesive formulation used is, it is generally suited to hot-melt coating techniques, which is advantageous in terms of environmental impact.

The following examples are the currently contemplated preferred modes for carrying out the invention and should not be considered as limiting thereof unless otherwise indicated.

EXAMPLES

In these examples, frontal tapes having a urethane release layer and fastening tapes utilizing various adhesives on the attaching surface were prepared, and they were attached through the application of pressure, after which a holding test as an index of the adhesion strength and an actual measurement of noise were carried out. Fastening tapes utilizing various adhesives on the bonding surface were also prepared and attached to a standard embossed diaper polyethylene after which a holding test and an adhesion value test were carried out.

In the production of the frontal tapes, a biaxially-oriented polypropylene film (BOPP) (thickness: 25 μm) was used as the substrate. A urethane release agent composition was applied on one side of the sample.

In the production of the fastening tapes, a non-oriented polypropylene film (CPP) (thickness: 100 μm) was used as the substrate, and adhesives shown by the samples A-H in Table 1 were coated from a toluene-heptane (4:1) solution on either the bonding surface or the attaching surface of the fastening tape by means of a knife coater to a thickness of 0.1 mm.

Table 1

|  | Tg | A | B | C | D | E | F | G | H | I | J |
|---|---|---|---|---|---|---|---|---|---|---|---|
| Kraton™ D 1107 | 215 | - | - | 40 | 30 | 30 | - | - | - | 56 | 47 |
| Kraton™ RP 6403 | 215 | 56 | 56 | - | - | - | 70 | 70 | 62 | - | - |
| Wingtack™ 95 | 323 | 32.7 | 38.7 | 46.9 | 57.1 | 58.7 | 7.1 | 14.0 | 19.9 | 32.7 | 38.8 |
| Wingtack™ 10 | 245 | 11.3 | 5.3 | 13.1 | 12.8 | 10.3 | 22.9 | 160 | 18.1 | 11.3 | 14.2 |
| Irganox™ 1076 | - | 1.0 | 1.0 | 1.0 | 1.0 | 1.0 | 1.0 | 1.0 | 1.0 | 1.0 | 1.0 |
| CMTg |  | 248 | 252 | 263 | 274 | 226 | 228 | 232 | 232 | 248 | 255 |

The adhesives A-J were coated onto the CPP substrate as shown in Table 2. Samples A, E, F, and J were comparative examples.

Table 2

|  | 1 | 2 | 3 | 4 | 5 | 6 | 7 | 8 | 9 |
|---|---|---|---|---|---|---|---|---|---|
| Bonding surface | A | B | C | D | E | C | C | C | C |
| Attaching Surface | H | H | H | H | H | F | G | I | J |

Wingtack™ 95 is a solid C5 tackifying resin available from Goodyear Chemical Co.

Irganox™ 1076 is an antioxidant available from Ciba Geigy Co.

The fastening tape bonding surface was attached to an embossed diaper polyethylene film and tested for the adhesion value and holding power. The fastening tape attaching surface was attached to the frontal tape and tested for holding power and noise level. The results of these tests are shown in Table 3.

Table 3

| Tape | Holding power to polyethylene (min) | Holding power to frontal tape (min) | Adhesion value to polyethylene (g/25mm) | Sound level (dB) |
|---|---|---|---|---|
| 1 | 50 | >120 | 1680 | 55.9 |
| 2 | >120 | >120 | 1840 | 55.9 |
| 3 | >120 | >120 | 2450 | 55.9 |
| 4 | >120 | >120 | 3150 | 55.9 |
| 5 | >120 | >120 | 1950 | 55.9 |
| 6 | >120 | 90 | 2450 | 53.3 |
| 7 | >120 | >120 | 2450 | 53.3 |
| 8 | >120 | >120 | 2450 | 59.2 |
| 9 | >120 | >120 | 2450 | 66.3 |

Adhesion value to Polyethylene film

A 35 $\mu$m thick polyethylene film was adhered to a steel panel with double coated tape. The bonding surface of a 25 mm wide fastening tape was placed on the polyethylene film and rolled down with one pass in each direction of a mechanically operated 2 kg roller. Peel strength of adhesive samples A-E were measured keeping the test angle at 180 degrees and the test peel speed at 300 mm/min.

Holding power to frontal tape

The frontal tape on the embossed polyethylene diaper backsheet was adhered to a steel panel. The fastening tape surface (test area 25 mm x 25 mm) was placed on the frontal tape or diaper backsheet and rolled down with one pass in each direction of a mechanically operated 2 kg roller. A 1 kg weight was hung vertically from the fastening tape at 40°C and holding time was measured in minutes.

Noise level

Disposable diapers were made of the fastening tape with a 25 x 45 mm area of the attaching surface adhered to the frontal tape and rolled down twice (once in each direction) with a 0.5 kg roller. The noise level was measured in dB at a peel speed of 500 mm/min.

It is clear from Table 3 that the adhesive tapes according to the present invention makes it possible to suppress the noise made on peeling off, while maintaining the adhesion strength between the frontal tape and the fastening tape and the fastening tape and the diaper polyethylene backsheet.

Examples 10-45

The samples were prepared by coating the adhesives as listed in Table 4 onto cast polypropylene (CPP) films, exhibiting a matte finish. For the examples, the polypropylene film was 4 mils (100 microns) thick and the adhesives were applied from a 50% solids solution in toluene and heptane (4 to 1 blend) in a conventional fashion. In all examples and counterexamples, the thickness of the adhesive is around 0.78 g (12 grains)/24 in$^2$ (1 inch = 2.54 cm) (50 microns). For each adhesive, 1% by weight of Irganox™ 1010, a hindered phenol antioxidant available from Ciba-Geigy, was added.

The resulting adhesives had the following compositions (the values in parentheses in Table 4 represent the percent of that particular component in the adhesive composition).

Table 4

| Ex. | CMTg | Elastomer(s) | Diblock Percent | Liquid Resin/Oil | Solid Resin |
|---|---|---|---|---|---|
| 10 | 254 | Kraton™1112 (52) | 40 | Shellflex™371 ( 2.9) | Wingtack Plus™ (45.1) |
| 12 | 245 | Kraton™1112 (52) | 40 | Shellflex™371 (11.2) | Wingtack Plus™ (36.8) |
| 13 | 264 | Kraton™1112 (30) | 40 | Shellflex™371 (12.8) | Wingtack Plus™ (57.2) |
| 14 | 261 | Kraton™1112 (35) | 40 | Shellflex™371 (11.2) | Wingtack Plus™ (53.8) |
| 15 | 259 | Kraton™1112 (39) | 40 | Shellflex™371 ( 9.5) | Wingtack Plus™ (51.5) |
| 16 | 256 | Kraton™1112 (43) | 40 | Shellflex™371 ( 8.8) | Wingtack Plus™ (48.2) |
| 17 | 252 | Kraton™1112 (47) | 40 | Shellflex™371 ( 9.0) | Wingtack Plus™ (44.0) |
| 18 | 247 | Kraton™1112 (56) | 40 | Shellflex™371 ( 5.9) | Wingtack Plus™ (38.1) |
| 19 | 244 | Kraton™1112 (60) | 40 | Shellflex™371 ( 5.2) | Wingtack Plus™ (34.8) |
| 20 | 247 | Kraton™1112 (38) | 40 | Shellflex™371 (21.4) | Wingtack Plus™ (40.6) |
| 21 | 246 | Kraton™1112 (45) | 40 | Shellflex™371 (16.3) | Wingtack Plus™ (38.7) |
| 22 | 250 | Kraton™1112 (42) | 40 | Shellflex™371 (15.1) | Wingtack Plus™ (42.9) |
| 23 | 255 | Kraton™1112 (35) | 40 | Shellflex™371 (16.5) | Wingtack Plus™ (48.5) |
| 24 | 253 | Kraton™1111 (26) / Kraton™1112 (26) | 27.5 | Shellflex™371 ( 4.7) | Wingtack Plus™ (43.3) |
| 25 | 244 | Kraton™1111 (26) / Kraton™1112 (26) | 27.5 | Shellflex™371 (12.9) | Wingtack Plus™ (34.1) |
| 26 | 253 | Kraton™1111 (26) / Shell RP6403 (26) | 35 | Shellflex™371 ( 4.7) | Wingtack Plus™ (43.3) |

Page 2 of Table 4

| Ex. | CMTg | Elastomer(s) | Diblock Percent | Liquid Resin/Oil | Solid Resin |
|---|---|---|---|---|---|
| 27 | 244 | Kraton™1111 (26) Shell RP6403 (26) | 35 | Shellflex™371 (12.9) | Wingtack Plus™ (34.1) |
| 28 | 254 | Kraton™1112 (52) | 40 | Shellflex™371 ( 2.9) | Wingtack Plus™ (45.1) |
| 29 | 245 | Kraton™1112 (52) | 40 | Shellflex™371 (11.2) | Wingtack Plus™ (36.8) |
| 30 | 253 | Kraton™1112 (26) Shell RP6403 (26) | 47.5 | Shellflex™371 ( 3.8) | Wingtack Plus™ (44.2) |
| 31 | 244 | Kraton™1112 (26) Shell RP6403 (26) | 47.5 | Shellflex™371 (12.2) | Wingtack Plus™ (35.8) |
| 32 | 245 | Shell RP6403 (52) | 55 | Shellflex™371 ( 2.9) | Wingtack Plus™ (45.1) |
| 33 | 254 | Shell RP6403 (52) | 55 | Shellflex™371 (11.2) | Wingtack Plus™ (36.8) |
| 34 | 254 | Kraton™1112 (52) | 40 | Shellflex™371 ( 2.9) | Wingtack Plus™ (45.1) |
| 35 | 245 | Kraton™1112 (52) | 40 | Shellflex™371 (11.2) | Wingtack Plus™ (36.8) |
| 36 | 254 | Kraton™1112 (52) | 40 | Zonarez™A-25 ( 5.0) | Escorez™1310 (43.0) |
| 37 | 245 | Kraton™1112 (52) | 40 | Zonarez™A-25 (21.8) | Escorez™1310 (26.2 |
| 38 | 254 | Kraton™1112 (52) | 40 | Shellflex™371 (12.7) | Zonarez™A-135 (35.3) |
| 39 | 245 | Kraton™1112 (52) | 40 | Shellflex™371 (19.2) | Zonarez™A-135 (28.8) |
| 40 | 254 | Kraton™1112 (52) | 40 | ECR™143-H ( 4.0) | Arkon™P100 (44.0) |
| 41 | 245 | Kraton™1112 (52) | 40 | ECR™143-H (19.9) | Arkon™P100 (28.1) |
| 42 | 254 | Kraton™1112 (52) | 40 | Escorez™2520 ( 5.1) | Escorez™1310 (42.9) |

EP 0 661 960 B1

Page 3 of Table 4

| Ex. | CMTg | Elastomer(s) | Diblock Percent | Liquid Resin/Oil | Solid Resin |
|-----|------|--------------|-----------------|------------------|-------------|
| 43 | 245 | Kraton™1112 (52) | 40 | Escorez™2520 (22.7) | Escorez™1310 (25.3) |
| 44 | 254 | Kraton™1112 (52) | 40 | Escorez™2520 ( 7.6) | Regalite™355 (40.4) |
| 45 | 245 | Kraton™1112 (52) | 40 | Escorez™2520 (24.2) | Regalite™355 (23.8) |

Counterexamples

| Ex. | CMTg | Elastomer(s) | Diblock Percent | Liquid Resin/Oil | Solid Resin |
|-----|------|--------------|-----------------|------------------|-------------|
| C1 | 254 | Kraton™1111 (52) | 15 | Shellflex™371 ( 4.7) | Wingtack Plus™ (43.3) |
| C2 | 245 | Kraton™1111 (52) | 15 | Shellflex™371 (12.7) | Wingtack Plus™ (35.3) |
| C3 | 254 | Kraton™1111 (52) | 15 | Shellflex™371 ( 4.7) | Wingtack Plus™ (43.3) |
| C4 | 245 | Kraton™1111 (52) | 15 | Shellflex™371 (12.7) | Wingtack Plus™ (35.3) |
| C5 | 263 | Kraton™1107 (33.5) | 15 | Zonarez™A-25 (20.0) | Escorez™1310 (46.5) |
| C6 | 263 | Kraton™1107 (39) | 15 | Wingtack™10 (10.8) | Wingtack Plus™ (50.2) |
| C7 | 257 | Kraton™1111 (40) | 15 | Shellflex™371 (12.0) | Wingtack Plus™ (48.0) |
| C8 | 255 | Kraton™1111 (38) | 15 | Shellflex™371 (15.3) | Wingtack Plus™ (46.7) |

EP 0 661 960 B1

Kraton™1107 is a polystyrene-isoprene linear block copolymer available from Shell Chemical Co. having a styrene/isoprene ratio of 14/86, approximately 15 to 20 percent diblock (A-B) and, 80 to 85 percent triblock (A-B-A) and a midblock Tg of 215 Kelvin.

Kraton™1111 is a polystyrene-isoprene linear block copolymer available from Shell Chemical Co. having a styrene content of about 21 percent, approximately 15 percent diblock and 85 percent triblock and a midblock Tg of 215 Kelvin.

Kraton™1112 is a polystyrene-isoprene linear block copolymer available from Shell Chemical Co. having a styrene content of about 14 percent, approximately 40 percent diblock and 60 percent triblock and a midblock Tg of 215 Kelvin.

Shell™RP6403 is a polystyrene-isoprene linear block copolymer available from Shell Chemical Co. having a styrene content of about 14 percent, approximately 55 percent diblock and 45 percent triblock and a midblock Tg of 215 Kelvin.

Escorez™1310 is a solid $C_5$ tackifying resin available from Exxon Chemical Corp. having a Tg of 313.5 Kelvin.

Wingtack™Plus is a solid $C_5$ tackifying resin with a Tg of 315 Kelvin available from Goodyear Chemical Co.

Wingtack™10 is a liquid $C_5$ hydrocarbon resin with a Tg of 245 Kelvin also from Goodyear Chemical Co.

Zonarez™A-25 is a liquid alpha pinene tackifying resin with a Tg of 251 Kelvin available from Arizona Chemical Co.

Zonarez™A-135 is a solid alpha pinene resin with a Tg of 367 Kelvin from Arizona Chemical Co.

Shellflex™371 is a naphthenic oil having about 10% aromatics measured by clay-gel analysis having a Tg of 209 Kelvin and is available from Shell Chemical Co.

ECR™143H is a hydrogenated aliphatic hydrocarbon resin with a Tg of 247 Kelvin available from Exxon Chemical Corp.

Escorez™2520 is a hydrogenated aliphatic hydrocarbon resin with a Tg of 253 Kelvin available from Exxon Chemical Corp.

Arkon™P100 is a hydrogenated $C_9$ tackifying resin with a Tg of 312.5 Kelvin available from Arakawa Chemical Co.

Regalite™355 is a hydrogenated rosin acid with a Tg of 318.1 Kelvin available from Hercules Inc.

The examples were then tested for their shear and 135° peel to a smooth frontal tape surface of biaxially oriented polypropylene (BOPP), and a matte finish cast polypropylene frontal tape, both having an LAB coating. The LAB was a copolymer of vinyl acetate and vinyl alcohol where some of the alcohol groups in the polymer backbone have been reacted with octadecyl isocyanate. The results are depicted in Table 5.

## Table 5

| Ex. | BOPP Shear | BOPP Peel | Shocky | Matte Shear | Matte Peel | Shocky |
|-----|------------|-----------|--------|-------------|------------|--------|
| C10 | 1400 | 287 | Yes | 1400 | 874 | No |
| 12 | 1400 | 461 | No | 220 | 586 | No |
| C13 | 450 | 213 | Yes | 120 | 662 | Yes |
| C14 | 1150 | 292 | Yes | 220 | 750 | S |
| 15 | 1400 | 342 | Yes | 700 | 714 | S |
| C16 | 1400 | 341 | Yes | 1210 | 656 | No |
| 17 | 1400 | 497 | S | 1040 | 540 | No |
| 18 | 1400 | 425 | No | 1400 | 464 | No |
| 19 | 1400 | 416 | No | 1100 | 390 | No |
| C20 | 40 | 285 | No | 10 | 457 | No |
| 21 | 500 | 471 | No | 76 | 534 | No |
| 22 | 570 | 569 | No | 140 | 619 | No |
| 23 | 240 | 629 | No | 105 | 693 | No |
| C24 | 1400 | 238 | Yes | 1400 | 846 | No |
| 25 | 1150 | 426 | No | 220 | 445 | No |

## Table 5 continued

| Ex. | Shear | Peel | Shocky | Shear | Peel | Shocky |
|-----|-------|------|--------|-------|------|--------|
| C26 | 1400 | 291 | S | 1400 | 920 | No |
| 27 | 990 | 450 | No | 350 | 509 | No |
| C28 | 1400 | 287 | Yes | 1400 | 874 | No |
| 29 | 1400 | 461 | No | 220 | 586 | No |
| 30 | 1400 | 529 | No | 1400 | 761 | No |
| 31 | 430 | 401 | No | 210 | 620 | No |
| 32 | 405 | 525 | No | 300 | 665 | No |
| 33 | 1400 | 380 | No | 1400 | 847 | No |
| C34 | 1400 | 287 | Yes | 1400 | 874 | No |
| 35 | 1400 | 461 | No | 220 | 586 | No |
| C36 | 1400 | 235 | Yes | 1400 | 825 | No |
| 37 | 370 | 415 | No | 100 | 552 | No |
| 38 | 1400 | 261 | Yes | 1400 | 699 | No |
| 39 | 80 | 463 | No | 16 | 372 | No |
| C40 | 1400 | 281 | Yes | 1400 | 828 | No |
| 41 | 810 | 467 | No | 400 | 548 | No |
| C42 | 1400 | 362 | S | 1400 | 814 | No |
| 43 | 260 | 402 | No | 30 | 541 | No |
| C44 | 1400 | 370 | S | 1400 | 476 | No |
| 45 | 405 | 460 | No | 40 | 268 | No |
| C1 | 1400 | 193 | Yes | 1400 | 872 | Yes |
| C2 | 1400 | 448 | S | 210 | 469 | Yes |
| C3 | 1400 | 168 | Yes | 1400 | 801 | Yes |
| C4 | 1400 | 468 | S | 390 | 443 | Yes |
| C5 | 1400 | 145 | Yes | 740 | 545 | Yes |
| C6 | 700 | 179 | Yes | 500 | 630 | Yes |
| C7 | 1400 | 233 | Yes | 1400 | 769 | Yes |
| C8 | 1400 | 432 | S | 60 | 502 | Yes |

S = Semi-shocky

Examples 10-23 demonstrate the effect of CMTg on the performance characteristics of the diaper attaching adhesive on the fastening tapes.

Examples 24 to 33 are examples of the effect of variance of the diblock content of the elastomeric component. No significant unexpected effects are noticed within the above defined area a when the diblock percentage is varied, with the noted exception of examples 30 and 33 which perform better than comparable examples with respect to their peel and noise performance. This indicates that for a higher diblock content adhesive elastomeric phase (e.g., above 45 percent) region a extends upward, to include region a'.

Examples C34 to 45 show the effect of varying the tackifying system from the preferred compounding oil and $C_5$ solid tackifier.

Examples C36 and 37 use a liquid alpha pinene resin instead of the oil. This creates a slight decrease in shear and peel performance (more-so shear) which is more noticeable at the lower CMTg formulation (more of the liquid alpha pinene resin).

Examples 38 and 39 use a solid alpha pinene resin. In this case the more noticeable performance degradation is at the lower CMTg formulation where unacceptable shear is encountered despite these formulations falling within region a.

Examples C36-39 appear to indicate that non-preferred alpha pinene and like higher aromatic tackifiers can be used, but possibly only in a blend with more preferred solid tackifiers at lower CMTg values within the preferred range.

Examples C40 and 41 use a liquid aliphatic tackifying resin and a solid hydrogenated $C_9$ tackifying resin. There is no significant performance variation with these particular tackifiers as compared to Examples C34 and 35.

Examples C42 and 43 use a liquid hydrogenated hydrocarbon resin and are comparable to examples 34 and 35. These examples exhibit better peel to smooth film at higher CMTg values yet unacceptable shear values to cast matte film.

Examples C44 and 45 also use a hydrogenated rosin acid, however, with no significant additional effects (compared to examples C42 and 43).

Counterexamples

Counterexamples 1 to 8 demonstrate the effect of lowering the diblock content of the elastomer component. The CMTg values for counterexamples 1 and 3 and 2 and 4 are substantially identical (the variations in properties are minor due to experimental error in measurements or lot variations in the raw materials) and are within the preferred CMTg ranges (254 and 245), yet the peel values are shocky or semi-shocky as compared to otherwise compositionally identical, e.g., examples 1 and 2, which provide non-shocky peels. This demonstrates the effectiveness of high diblock content in reducing shocky peels, while still providing elastomeric compositions with adequate shear performance.

Examples 46-59

These examples were prepared, as with Examples 10-45, from a solution coated onto a CPP film. The adhesive thickness was about 0.78 g (12 grains)/24 in$^2$ (1 inch = 2.54 cm) (about 50 microns). The resulting adhesives had the compositions set forth in Table 6 below in percent by weight.

Table 6

| EXAMPLE | CMTg | ELASTOMER[1] | LIQUID RESIN | SOLID RESIN |
|---|---|---|---|---|
| 46 | 241 | 42 | $49.2^2$ | $8.8^4$ |
| 47 | 241 | 53 | $29.1^2$ | $17.9^4$ |
| 48 | 241 | 58 | $20.0^2$ | $22.0^4$ |
| 49 | 241 | 67 | $3.6^2$ | $29.4^4$ |
| 50 | 235 | 58 | $32.8^2$ | $9.2^4$ |
| 51 | 235 | 67 | $16.2^2$ | $16.8^4$ |
| 52 | 230 | 67 | $27.2^2$ | $5.8^4$ |
| 53 | 241 | 53 | $25.6^3$ | $21.4^5$ |
| 54 | 241 | 58 | $17.5^3$ | $24.5^5$ |
| 55 | 241 | 67 | $2.9^3$ | $30.1^5$ |
| 56 | 235 | 43 | $53.6^3$ | $3.4^5$ |
| 57 | 235 | 53 | $37.2^3$ | $9.8^5$ |
| 58 | 235 | 67 | $14.2^3$ | $18.8^5$ |
| 59 | 230 | 67 | $28.1^3$ | $4.9^5$ |

1. Kraton™ 1112
2. Escorez™ 2520
3. ECR™ 143H
4. Escorez™ 1310
5. Arkon™ P-100

These tapes were then tested for 135° peel and shear performance to the same BOPP and cast polypropylene films as Examples 10-45. The results are depicted in Table 7. All the peels were non-shocky.

Table 7

| EXAMPLE | BOPP SHEAR | BOPP PEEL | MATTE SHEAR | MATTE PEEL |
|---|---|---|---|---|
| 46 | 10 | 147 | 10 | 198 |
| 47 | 96 | 323 | 11 | 200 |
| 48 | 224 | 330 | 29 | 260 |
| 49 | 1400 | 349 | 1400 | 338 |
| 50 | 26 | 259 | 10 | 158 |
| 51 | 386 | 258 | 61 | 196 |
| 52 | 111 | 147 | 10 | 49 |
| 53 | 213 | 279 | 20 | 230 |
| 54 | 471 | 342 | 129 | 220 |
| 55 | 1400 | 228 | 1400 | 170 |
| 56 | 11 | 216 | 10 | 170 |
| 57 | 61 | 279 | 10 | 172 |
| 58 | 316 | 207 | 23 | 145 |
| 59 | 68 | 92 | 10 | 26 |

In this lower CMTg range, the peel performance to the BOPP and cast matte films was lower than for Examples 10-45, which had higher CMTg values. However, the peel performance was still functional and non-shocky.

Examples 60-66

These tapes were hot-melt coated onto a backing at a coating weight of about 0.78 g (12 grains)/24 in$^2$ 1 inch = 2.54 cm (50 microns). The adhesive formulations are given below in Table 8 in percent by weight.

Table 8

| EXAMPLE | CMTg | ELASTOMER[1] | PLASTICIZER[2] | SOLID RESIN[3] |
|---|---|---|---|---|
| 60 | 244 | 47 | 17.8 | 35.2 |
| 61 | 244 | 50 | 15.3 | 34.7 |
| 62 | 244 | 53 | 12.8 | 34.2 |
| 63 | 244 | 56 | 10.3 | 33.7 |
| 64 | 241 | 50 | 18.1 | 31.9 |
| 65 | 241 | 53 | 15.6 | 31.4 |
| 66 | 241 | 56 | 13.0 | 31.0 |

1. RP 6411- a polyisoprene-polystyrene block copolymer with a midblock Tg of about 215 Kelvin, approximately 65 percent diblock and 35 percent triblock, available from Shell Chemical Co.
2. Shellflex™ 371
3. Wingtack™ Plus

These tapes were then tested for 135° peel and shear performance, as per the previous examples, except that the tapes were tested against a standard embossed diaper polyethylene film instead of the matte cast polypropylene. The results are given in Table 9 below.

Table 9

| EXAMPLE | SHEAR BOPP | BOPP PEEL | POLY SHEAR | POLY PEEL |
|---|---|---|---|---|
| 60 | 211 | 602 | 59 | 638 |
| 61 | 266 | 630 | 70 | 724 |
| 62 | 573 | 685 | 332 | 775 |
| 63 | 1176 | 633 | 794 | 852 |
| 64 | 263 | 575 | 63 | 578 |
| 65 | 263 | 624 | 59 | 670 |
| 66 | 388 | 630 | 108 | 668 |

All the tapes exhibited substantially non-shocky peel performance. However, a problem with these adhesives, particularly at the lower polymer levels, e.g., less than about 50 percent at 244 Kelvin and less than about 60 percent at 241 Kelvin and below, is that the adhesives exhibit poor shear adherence to diaper polyethylene, including the need for the separate bonding adhesive.

Example 67

The tape of Example 66 was tested for 135 degree peel and shear performance against a BOPP frontal tape coated with an organopolysiloxane-polyurea block copolymer comprising the condensation reaction of an organopolysiloxane diamine with a diisocyanate and a diamine chain extender. The silicone diamine segment had a molecular weight of about 50,000 and was prepared such as described in EP-A-250 248 and EP-A-380 236. The silicone diamine was then reacted with "Jeffamine™" DU 700 (available from Texaco Co.), 1,3 diaminopentane, and isophorone diisocyanate, to provide a polymer with about 6 weight percent silicone, 34 weight percent "Jeffamine™" soft segment and 60 weight percent hard segment. The 135 degree peel value was 66 grams/inch (168 grams/cm) and the peel was smooth. The shear value was 637 minutes.

Test Methods

135 Degree Peel from Frontal Tape

The peel adhesion test is a 135 degree peel from a smooth frontal tape surface of biaxially oriented polypropylene(BOPP) or a matte cast polypropylene, having a LAB on it. The peel rate is 30.5 cm (12 inches) per minute. The tape samples are rolled down onto the frontal tape substrate using two passes of a 4.5 pound roller. This test is a variation on PSTC-5. The data is reported in grams per inch and was run at 21° C (70° F) and 50 percent relative humidity.

Shear from Frontal Tape Substrate

The shear adhesion is measured by determining the length of time it takes for a 1 inch by 1 inch (1 inch = 2.54 cm) sample to shear off a frontal tape substrate under a 1 kilogram load. The frontal tape is either a smooth frontal tape(BOPP) with a LAB as described above or a matte polypropylene(PP) with an LAB. The 2" by 6" piece of frontal tape is laminated to a 2" by 6" (1" = 2.54 cm) piece of reinforcing tape (DPDY-9377) in order to enhance the stiffness of the substrate. On the side opposite the reinforcing tape, a one by two inch area of the test tape is rolled down onto the frontal tape using 2 passes of a 2 kg (4.5 pound) roller. The overlap area between the test tape and the substrate is one by one inch. The laminated substrate and the test tape are hung vertically in a 40°C oven for 15 minutes after which a 1 kilogram weight is hung from the test tape, generating a shear load at a 180° angle. The time in minutes for the weight to drop is used as the measure of the shear adhesion.

Other embodiments of the invention will be apparent to those skilled in the art from the consideration of the specification of or practice of the invention disclosed herein. It is intended that the specifications and examples be considered as exemplary, with the true scope of the invention being indicated by the following claims.

**Claims**

1. A diaper tape closure system comprising a disposable diaper (1) having a first closure surface (4) comprising a reinforcement frontal film, the frontal film having a first inner face and a second outer face having a low adhesion backsize (LAB) coating (8), a second closure surface comprising a fastening tape tab (5) having a backing substrate (6) with an attaching surface (13) and a bonding surface (12), said bonding surface (12) having a bonding adhesive layer (11) thereon and said attaching surface (13) having an attaching adhesive layer (10) for attachment to said first closure surface (4) LAB coated second outer face (8), said fastening tab attaching adhesive layer (10) characterized in that said attaching adhesive comprises an admixture of an A-B type block copolymer having at least 15 percent diblock copolymer and a tackifier with the glass transition point of the adhesive admixture being in the range of 230K to 265K, and the adhesive admixture further falling within region a or a' of Fig. 3 and the bonding adhesive layer (11) comprises an adhesive falling within region b of Fig. 4 and also comprises an admixture of an A-B type block copolymer and tackifier.

2. A disposable diaper closure system according to Claim 1, wherein said A-B type block copolymer B block is derived primarily from isoprene and where the attaching adhesive admixture falls within region a of Fig. 3.

3. A disposable diaper closure system according to Claim 2, wherein the bonding adhesive is one falling within region c of Fig. 4.

4. A disposable diaper closure system according to any one of Claims 1 to 3, wherein said attaching adhesive and/or bonding adhesive A-B type block copolymers are a mixture of polymers represented by the following general formulae:

$$A\text{-}(B\text{-}A)n \text{ and } A\text{-}B$$

wherein A is a vinyl aromatic hydrocarbon, B is derived primarily from isoprene, and n is an integer of one or more, and said tackifier is a rosin type, terpene type, aliphatic petroleum hydrocarbon resin (C5) type, or an aromatic petroleum hydrocarbon resin (C9) type tackifier, or a hydrogenated product thereof.

5. A diaper tape closure system according to Claim 1, wherein said attaching adhesive layer (10) is further characterized in that it comprises:

    (a) 30 to 60 weight percent of an elastomer component comprised of a block copolymer with A blocks derived primarily from styrene and B blocks derived primarily from isoprene wherein at least 25 percent of the block copolymer is in the form of an AB diblock copolymer,
    (b) 40 to 70 weight percent of a solid B block compatible tackifying resin admixed when necessary with a liquid tackifying resin or a processing oil so as to provide an adhesive composition having a CMTg of from 244 to 265 Kelvin which attaching adhesive layer (10) on said fastening tape tab (5) exhibits a shear adhesion of at least about 200 minutes to the LAB coated film backing (9) face and a substantially nonshocky peel at 135 degrees of at least about 350 grams per 2.54 cm (inch), and the bonding adhesive also comprises an A-B type block copolymer with the A and B blocks defined as above.

6. A diaper tape closure system according to any one of Claims 1-4, wherein said attaching adhesive layer (10) is further characterized in that it comprises:

    (a) 45 to 95 weight percent of an elastomer component comprised of a block copolymer with A blocks derived primarily from styrene and B blocks derived primarily from isoprene wherein at least 15 percent of the block copolymer is in the form of an AB diblock copolymer,
    (b) 5 to 55 weight percent of a solid B block compatible tackifying resin admixed when necessary with a liquid tackifying resin or a processing oil so as to provide an adhesive composition having a CMTg of from 230 to 244 Kelvin which attaching adhesive layer (10) on said fastening tape tab (5) exhibits a shear adhesion of at least about 100 minutes to the LAB coated (8) film backing (9) face and a substantially nonshocky peel at 135 degrees of at least about 200 grams per 2.54 cm (inch) said attaching adhesive falling within region a of Fig. 3.

7. The tape closure system of Claims 5 or 6 wherein the elastomer component further comprises an A-B-A block copolymer.

8. The tape closure system of Claim 6 wherein the tape exhibits a peel at 135 degrees of at least about 250 grams per 2.54 cm (inch), the bonding adhesive falling within region <u>b</u> of Fig. 4, the bonding adhesive also comprising an A-B type block copolymer and tackifier with the A and B blocks defined as above.

9.  The tape closure system of Claims 5 or 6 wherein the LAB coated film (9) has a smooth surface on the LAB coated face.

10. The tape closure system of any of Claims 1-9 wherein the LAB coating (8) is a urethane.

11. The tape closure system of any of Claims 1-9 wherein the LAB coating (8) comprises 100 parts silicone release agent and 20-200 parts release-controlling agent with an appropriate catalyst.

12. The tape closure system of any of Claims 1-9 wherein the LAB coating (8) comprises an organopolysiloxane-polyurea with a silicone diamine soft segment of at least 2,000 molecular weight and comprising at least 1 weight percent of the LAB coating (8).

13. The tape closure of Claim 12 wherein the LAB coating (8) comprises a silicone diamine with a molecular weight of from 2,000 to 50,000 and from 2 to 15 weight percent of the release coating, from 20 to 40 weight percent non-silicone soft segment and from 40 to 80 weight percent diisocyanate hard segment.

14. The tape closure system of Claim 1 wherein said disposable diaper (1) has an outer backsheet face (2) formed of a polyethylene polymer or copolymer film or a nonwoven, and said bonding adhesive layer (11) is permanently bonded to said outer backsheet face (2).

15. The tape closure system of Claim 14 wherein the percent diblock of the AB type block copolymer comprises at least 25 percent diblock copolymer.

16. The tape closure system of Claim 15, wherein said attaching adhesive layer (10) is further characterized in that it comprises:

    (a) 30 to 60 weight percent of an elastomer component comprised of said A-B type block copolymer with A blocks derived primarily from styrene wherein at least 30 to 95 percent of the block copolymer is in the form of an AB diblock copolymer.
    (b) 40 to 70 weight percent of a solid B block compatible tackifying resin admixed when necessary with a liquid tackifying resin or a processing oil so as to provide an adhesive composition having a CMTg of from 244 to 265 Kelvin which attaching adhesive layer on said fastening tape exhibits a shear adhesion of at least about 200 minutes to the LAB coated film backing face and also comprises an A-B type block copolymer and tackifier, with the A and B blocks defined as above.

17. The tape closure system of Claim 16 wherein the elastomer component further comprises an A-B-A block copolymer.

**Patentansprüche**

1.  Verschlußstreifensystem für eine Windel, umfassend eine Wegwerfwindel (1) mit einer ersten Verschlußoberfläche (4), umfassend eine vordere Verstärkungsfolie, wobei die vordere Folie eine erste innere Seite und eine zweite äußere Seite mit einer schwach klebenden Gegenstück (low adhesion backsize LAB)-Beschichtung (8) aufweist, und einer zweiten Verschlußoberfläche, umfassend ein Befestigungsstreifenstück (5), das ein Trägermaterial (6) mit einer Anbringoberfläche (13) und einer Bindungsoberfläche (12) aufweist, wobei auf der Bindungsoberfläche (12) eine Bindungsklebeschicht (11) und auf der Anbringoberfläche (13) eine Anbringklebeschicht (10) zum Anbringen auf der zweiten äußeren LAB-beschichteten Seite (8) der ersten Verschlußoberfläche (4) ist, wobei die Anbringklebeschicht (10) des Befestigungsstücks dadurch gekennzeichnet ist, daß der Anbringklebstoff ein Gemisch eines Blockcopolymers vom A-B-Typ mit mindestens 15 % Zweiblockcopolymer und eines Klebrigmachers umfaßt, wobei die Glasübergangstemperatur des Klebstoffgemisches im Bereich von 230 K bis 265 K liegt und das Klebstoffgemisch ferner in das Gebiet <u>a</u> oder <u>a</u>' der Figur 3 fällt und die Bindungsklebeschicht (11) einen Klebstoff umfaßt, der in das Gebiet <u>b</u> der Figur 4 fällt und auch ein Gemisch eines Blockcopolymers vom A-B-Typ und einen Klebrigmacher umfaßt.

2.  Verschlußsystem für eine Wegwerfwindel gemäß Anspruch 1, wobei sich der B-Block des Blockcopolymers vom A-B-Typ vor allem aus Isopren ableitet und das Anbringklebstoffgemisch in das Gebiet <u>a</u> der Figur 3 fällt.

3. Verschlußsystem für eine Wegwerfwindel gemäß Anspruch 2, wobei der Bindungsklebstoff in das Gebiet c der Figur 4 fällt.

4. Verschlußsystem für eine Wegwerfwindel gemäß einem der Ansprüche 1 bis 3, wobei die Anbringklebstoff- und/oder Bindungsklebstoff-Blockcopolymere vom A-B-Typ ein Gemisch von Polymeren der folgenden allgemeinen Formel sind:

A-(B-A)n und A-B

worin A ein vinyl-aromatischer Kohlenwasserstoff ist, B sich vor allem aus Isopren ableitet und n eine ganze Zahl von 1 oder mehr ist und der Klebrigmacher ein harzartiger, ein terpenartiger, ein aliphatisch ölkohlenwasserstoffharz (C5)-artiger oder ein aromatisch ölkohlenwasserstoffharz (C9)-artiger Klebrigmacher oder ein hydriertes Produkt davon ist.

5. Verschlußsystem für eine Windel gemäß Anspruch 1, worin die Anbringklebeschicht (10) ferner dadurch gekennzeichnet ist, daß sie umfaßt:

(a) 30 bis 60 Gew.-% einer Elastomerkomponente umfassend ein Blockcopolymer mit A-Blöcken, die sich vor allem aus Styrol ableiten, und B-Blöcken, die sich vor allem aus Isopren ableiten, wobei wenigstens 25 % des Blockcopolymers in der Form eines AB-Zweiblock-Copolymers vorliegen,
(b) 40 bis 70 Gew.-% eines festen mit dem B-Block verträglichen Klebrigmacherharzes, das, wenn notwendig, mit einem flüssigen Klebrigmacherharz oder einem Weichmacheröl vermischt ist, um eine Klebezusammensetzung mit einem CMTg von 244 bis 265 Kelvin bereitzustellen, wobei die Anbringklebeschicht (10) auf dem Befestigungsstreifenstück (5) ein Scherhaftvermögen von wenigstens etwa 200 Minuten an der LAB-beschichteten Folienträgerseite (9) und ein im wesentlichen nicht abruptes Ablösen bei 135° und wenigstens etwa 350 g pro 2,54 cm (Inch) zeigt und der Bindungsklebestoff auch ein Blockcopolymer vom A-B-Typ umfaßt, wobei die A- und B-Blöcke wie vorstehend definiert sind.

6. Verschlußsystem für eine Windel gemäß einem der Ansprüche 1 bis 4, wobei die Anbringklebeschicht (10) ferner dadurch gekennzeichnet ist, daß sie umfaßt:

(a) 45 bis 95 Gew.-% einer Elastomerkomponente umfassend ein Blockcopolymer mit A-Blöcken, die sich vor allem aus Styrol ableiten, und B-Blöcken, die sich vor allem aus Isopren ableiten, wobei wenigstens 15 % des Blockcopolymers in der Form eines AB-Zweiblock-Copolymers vorliegen,
(b) 5 bis 55 Gew.-% eines festen mit dem B-Block verträglichen Klebrigmacherharzes, das, wenn notwendig, mit einem flüssigen Klebrigmacherharz oder einem Weichmacheröl vermischt ist, um eine Klebezusammensetzung mit einem CMTg von 230 bis 244 Kelvin bereitzustellen, wobei die Anbringklebeschicht (10) auf dem Befestigungsstreifenstück (5) ein Scherhaftvermögen von wenigstens etwa 100 Minuten an der LAB-beschichteten (8) Folienträgerseite (9) und ein im wesentlichen nicht abruptes Ablösen bei 135° und wenigstens etwa 200 g pro 2,54 cm (Inch) zeigt, wobei der Anbringklebstoff in das Gebiet a der Figur 3 fällt.

7. Verschlußstreifensystem gemäß den Ansprüchen 5 oder 6, wobei die Elastomerkomponente ferner ein A-B-A-Blockcopolymer umfaßt.

8. Verschlußstreifensystem gemäß Anspruch 6, wobei der Streifen ein Ablösen bei 135° und wenigstens etwa 250 g pro 2,54 cm (Inch) zeigt, der Bindungsklebstoff in das Gebiet b der Figur 4 fällt und auch ein Blockcopolymer vom A-B-Typ und einen Klebrigmacher umfaßt, wobei die A- und B-Blöcke wie vorstehend definiert sind.

9. Verschlußstreifensystem gemäß den Ansprüchen 5 oder 6, wobei die LAB-beschichtete Folie (9) eine glatte Oberfläche auf der LAB-beschichteten Seite hat.

10. Verschlußstreifensystem gemäß einem der Ansprüche 1 bis 9, wobei die LAB-Beschichtung (8) ein Urethan ist.

11. Verschlußstreifensystem gemäß einem der Ansprüche 1 bis 9, wobei die LAB-Beschichtung (8) 100 Teile eines Silikonablösemittels und 20 bis 200 Teile eines die Ablösung kontrollierenden Mittels mit einem geeigneten Katalysator umfaßt.

12. Verschlußstreifensystem gemäß einem der Ansprüche 1 bis 9, wobei die LAB-Beschichtung (8) einen Organopolysiloxan-Polyharnstoff mit einem Silikondiaminsoftsegment umfaßt, das ein Molekulargewicht von wenigstens 2000 aufweist und wenigstens 1 Gew.-% der LAB-Beschichtung (8) umfaßt.

13. Verschlußstreifensystem gemäß Anspruch 12, wobei die LAB-Beschichtung (8) ein Silikondiamin mit einem Molekulargewicht von 2000 bis 50 000, 2 bis 15 Gew.-% der Ablösebeschichtung, 20 bis 40 Gew.-% eines nicht-silikonhaltigen Softsegments und 40 bis 80 Gew.-% eines Diisocyanathartsegments umfaßt.

14. Verschlußstreifensystem gemäß Anspruch 1, wobei die Wegwerfwindel (1) eine äußere rückseitige Seite (2) aus einer Polyethylenpolymer- oder Copolymerfolie oder eines Faservlieses aufweist, und die Bindungsklebeschicht (11) dauerhaft an der äußeren rückseitigen Seite (2) haftet.

15. Verschlußstreifensystem gemäß Anspruch 14, wobei der Zweiblockanteil im Blockcopolymer vom A-B-Typ wenigstens 25 % Zweiblockcopolymer umfaßt.

16. Verschlußstreifensystem gemäß Anspruch 15, wobei die Anbringklebeschicht (10) ferner dadurch gekennzeichnet ist, daß sie umfaßt:

(a) 30 bis 60 Gew.-% einer Elastomerkomponente, umfassend ein Blockcopolymer vom A-B-Typ, wobei sich die A-Blöcke vor allem aus Styrol ableiten, wobei wenigstens 30 bis 95 % des Blockcopolymers in der Form eines A-B-Zweiblockcopolymers vorliegen,
(b) 40 bis 70 Gew.-% eines festen mit dem B-Block verträglichen Klebrigmacherharzes, das, wenn notwendig, mit einem flüssigen Klebrigmacherharz oder einem Weichmacheröl vermischt ist, um eine Klebezusammensetzung mit einem CMTg von 244 bis 265 Kelvin bereitzustellen, wobei die Anbringklebeschicht auf dem Befestigungsstreifenstück ein Scherhaftvermögen von wenigstens etwa 200 Minuten an der LAB-beschichteten Folienträgerseite zeigt und ferner ein Blockcopolymer vom A-B-Typ und einen Klebrigmacher umfaßt, wobei die A- und B-Blöcke wie vorstehend definiert sind.

17. Verschlußstreifensystem gemäß Anspruch 16, wobei die Elastomerkomponente ferner ein A-B-A-Blockcopolymer umfaßt.

**Revendications**

1. Système de fermeture en ruban pour couche comprenant une couche jetable (1) comportant une première surface de fermeture (4) comprenant un film frontal de renforcement, le film frontal comportant une première face intérieure et une seconde face extérieure comportant un revêtement (8) d'apprêt de faible adhérence (LAB), une seconde surface de fermeture comprenant une patte en ruban de fixation (5) comportant un substrat de support (6) avec une surface d'attache (13) et une surface de collage (12), la surface de collage (12) comportant une couche d'adhésif de collage (11) et la surface d'attache (13) comportant une couche d'adhésif d'attache (10) pour l'attache à la seconde face extérieure revêtue de LAB (8) de la première surface de fermeture (4), la patte de fixation attachant la couche d'adhésif (10), caractérisé en ce que l'adhésif d'attache comprend un mélange d'un copolymère bloc du type A-B comportant au moins 15 % de copolymère dibloc et une colle, le point de transition vitreuse du mélange adhésif se situant dans la gamme de 230K à 265K et le mélange adhésif tombant de plus dans la zone a ou a' de la figure 3, et la couche d'adhésif de collage (11) comprend un adhésif tombant dans la zone b de la figure 4 et comprend également un mélange d'un copolymère bloc du type A-B et de colle.

2. Système de fermeture pour couche jetable suivant la revendication 1, dans lequel le bloc B du copolymère bloc du type A-B provient principalement d'isoprène et dans lequel le mélange adhésif d'attache tombe dans la zone a de la figure 3.

3. Système de fermeture pour couche jetable suivant la revendication 2, dans lequel l'adhésif de collage est un adhésif tombant dans la zone c de la figure 4.

4. Système de fermeture pour couche jetable suivant l'une quelconque des revendications 1 à 3, dans lequel les copolymères blocs du type A-B de l'adhésif d'attache et/ou de l'adhésif de collage sont un mélange de polymères représentés par les formules générales suivantes :

A-(B-A)n et A-B

dans lesquelles A est un hydrocarbure aromatique vinylique, B provient principalement d'isoprène et n est un nombre entier de 1 ou plus et la colle est une colle du type colophane, du type terpène, du type résine hydrocarbonée de pétrole aliphatique (C5) ou du type résine hydrocarbonée de pétrole aromatique (C9) ou un produit hydrogéné de ceux-ci.

5. Système de fermeture en ruban pour couche suivant la revendication 1, dans lequel la couche d'adhésif d'attache (10) précitée est de plus caractérisée en ce qu'elle comprend :

(a) 30 à 60 % en poids d'un composant élastomère constitué d'un copolymère bloc avec des blocs A provenant principalement de styrène et des blocs B provenant principalement d'isoprène dans lequel au moins 25 % du copolymère bloc sont sous la forme d'un copolymère dibloc AB,
(b) 40 à 70 % en poids d'une résine solide assurant un collage compatible avec les blocs B mélangée en fonction des nécessités avec une résine liquide assurant un collage ou une huile de traitement de manière à former une composition adhésive ayant une CMTg de 244 à 265 Kelvins, laquelle couche d'adhésif d'attache (10) sur la patte en ruban de fixation (5) montre une adhérence au cisaillement d'au moins environ 200 minutes à la face du support de film (9) revêtue de LAB et un pelage essentiellement non saccadé à 135 degrés d'au moins environ 350 grammes par 2,54 cm (pouce) et l'adhésif de collage comprend également un copolymère bloc du type A-B avec les blocs A et B tels que définis ci-dessus.

6. Système de fermeture en ruban pour couche suivant l'une quelconque des revendications 1 à 4, dans lequel la couche d'adhésif d'attache (10) précitée est de plus caractérisée en ce qu'elle comprend :

(a) 45 à 95 % en poids d'un composant élastomère constitué d'un copolymère bloc avec des blocs A provenant principalement de styrène et des blocs B provenant principalement d'isoprène dans lequel au moins 15 % du copolymère bloc sont sous la forme d'un copolymère dibloc AB,
(b) 5 à 55 % en poids d'une résine solide assurant un collage compatible avec les blocs B mélangée en fonction des nécessités avec une résine liquide assurant un collage ou une huile de traitement de manière à former une composition adhésive ayant une CMTg de 230 à 244 Kelvins, laquelle couche d'adhésif d'attache (10) sur la patte en ruban de fixation (5) montre une adhérence au cisaillement d'au moins environ 100 minutes à la face du support de film (9) revêtue de LAB (8) et un pelage essentiellement non saccadé à 135 degrés d'au moins environ 200 grammes par 2,54 cm (pouce), l'adhésif d'attache tombant dans la zone a de la figure 3.

7. Système de fermeture en ruban suivant l'une ou l'autre des revendications 5 et 6, dans lequel le composant élastomère comprend de plus un copolymère bloc A-B-A.

8. Système de fermeture en ruban suivant la revendication 6, dans lequel le ruban montre un pelage à 135 degrés d'au moins environ 250 grammes par 2,54 cm (pouce), l'adhésif de collage tombant dans la zone b de la figure 4, l'adhésif de collage comprenant également un copolymère bloc du type A-B et de la colle, les blocs A et B étant tels que définis précédemment.

9. Système de fermeture en ruban suivant l'une ou l'autre des revendications 5 et 6, dans lequel le film revêtu de LAB (9) comporte une surface lisse sur la face revêtue de LAB.

10. Système de fermeture en ruban suivant l'une quelconque des revendications 1 à 9, dans lequel le revêtement de LAB (8) est un uréthanne.

11. Système de fermeture en ruban suivant l'une quelconque des revendications 1 à 9, dans lequel le revêtement de LAB (8) comprend 100 parties d'agent de séparation à base de silicone et 20-200 parties d'agent de contrôle de séparation avec un catalyseur approprié.

12. Système de fermeture en ruban suivant l'une quelconque des revendications 1 à 9, dans lequel le revêtement de LAB (8) comprend une organopolysiloxane-polyurée avec un segment souple de silicone diamine d'au moins 2.000 de poids moléculaire et comprenant au moins 1 % en poids du revêtement de LAB (8).

13. Fermeture en ruban suivant la revendication 12, dans laquelle le revêtement de LAB (8) comprend une silicone diamine d'un poids moléculaire de 2.000 à 50.000 et de 2 à 15 % en poids du revêtement de séparation, de 20 à 40 % en poids de segment souple non siliconé et de 40 à 80% en poids de segment dur de diisocyanate.

14. Système de fermeture en ruban suivant la revendication 1, dans lequel la couche jetable (1) précitée comporte une face de feuille de support extérieure (2) formée d'un film de polymère ou de copolymère de polyéthylène ou d'un non-tissé et la couche d'adhésif de collage (11) susdite est liée de façon permanente à ladite face de feuille de sup-

22

port extérieure (2).

**15.** Système de fermeture en ruban suivant la revendication 14, dans lequel le pourcentage en diblocs du copolymère bloc du type AB constitue au moins 25 % du copolymère dibloc.

**16.** Système de fermeture en ruban suivant la revendication 15, dans lequel la couche d'adhésif d'attache (10) est de plus caractérisée en ce qu'elle comprend :

(a) 30 à 60 % en poids d'un composant élastomère constitué du copolymère bloc du type A-B précité avec des blocs A provenant principalement de styrène dans lequel au moins 30 à 95 % du copolymère bloc sont sous la forme d'un copolymère dibloc AB,
(b) 40 à 70 % en poids d'une résine solide assurant un collage compatible avec les blocs B mélangée en fonction des nécessités avec une résine liquide assurant un collage ou une huile de traitement de manière à former une composition adhésive ayant une CMTg de 244 à 265 Kelvins, laquelle couche d'adhésif d'attache sur le ruban de fixation montre une adhérence au cisaillement d'au moins environ 200 minutes à la face du support de film revêtue de LAB et comprend également un copolymère bloc du type A-B et une colle, les blocs A et B étant tels que définis précédemment.

**17.** Système de fermeture en ruban suivant la revendication 16, dans lequel le composant élastomère comprend de plus un copolymère bloc A-B-A.

FIG. 1

FIG. 2

FIG. 3

FIG. 4